# EUROPEAN PATENT APPLICATION

(11) **EP 4 413 991 A1**
(43) Date of publication of application: **14.08.2024**
(21) Application number: 23382113.1
(22) Date of filing: 08.02.2023
(51) Int. Cl.: A61K 38/02, A61K 38/04, A61P 25/28

(54) **PEPTIDE FOR COGNITIVE IMPROVEMENT**

(71) Applicant: Consejo Superior De Investigaciones Científicas, 28006 Madrid (ES); Universidad Autónoma de Madrid, 28049 Madrid (ES)
(72) Inventor: Avila de Grado, Jesús, Madrid (ES); Antón Fernández, Alejandro, Madrid (ES); Hernández Pérez, Félix, Madrid (ES)
(74) Representative: Pons

(57) **Abstract**

The present invention relates to a peptide comprising SEQ ID NO: 1, or a composition comprising said peptide, for use in the treatment and/or prevention of a cognitive disorder in a subject. It also relates to the non-therapeutic use of said peptide, and a related method, for the cognitive function amelioration.

## Description

The invention relates to uses and methods for the treatment and/or prevention of cognitive disorders, as well as for the cognitive function improvement. Thus, the present invention belongs to the field of neurosciences and neurobiology.

### BACKGROUND ART

Impairments in cognitive function can occur in different conditions or diseases. In fact, along with rapid global population aging, the age-related cognitive disorders have posed a serious threat to public health, health care system, and sustainable economic and societal development of all countries.

Furthermore, modern society is characterized by an increased focus on academic achievement as well as by environments which demand high cognitive capabilities.

Different strategies to improve cognitive function and/or to treat disorders related to the central nervous system are described in the state of the art:
US2019111059A1 discloses the use of reduced folates to treat disorders and symptoms related to the central nervous system. In particular, said document provides means and methods for administering folates to the central nervous system, when the folate receptor alpha (FRα) system is impaired, and describes that the administration of reduced folates can correct underlying folate deficiencies in the central nervous system, which in turn can result in an improvement of one or more symptoms of central nervous system disorders.
WO2022097136A1 provides a method for preventing, treating, ameliorating or delaying the onset of neurodegenerative diseases, cognitive decline and any conditions or symptoms associated therewith in a subject in need thereof, comprising administering to said subject an effective amount of an isolated polypeptide.

In view of the state of the art, there is a need to identify alternative compounds for the prevention and/or treatment of cognitive disorders, as well as for the amelioration of cognitive function, even more so given the ever-increasing ageing of the population.

### DESCRIPTION OF THE INVENTION

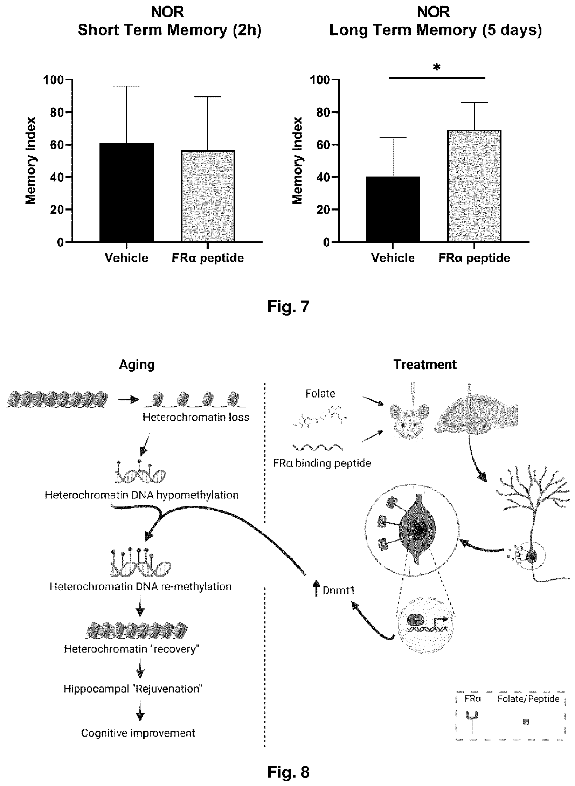

Inventors have demonstrated that a peptide which is able to bind to folate receptor α (FRα), comprising amino acid sequence SEQ ID NO: 1 (which is also referred hereinafter as "FRα binding peptide", "FRalpha peptide", "FRα peptide", or as the "peptide of the invention"), led to a significant improvement of long-term memory in aged mice (Fig. 7).

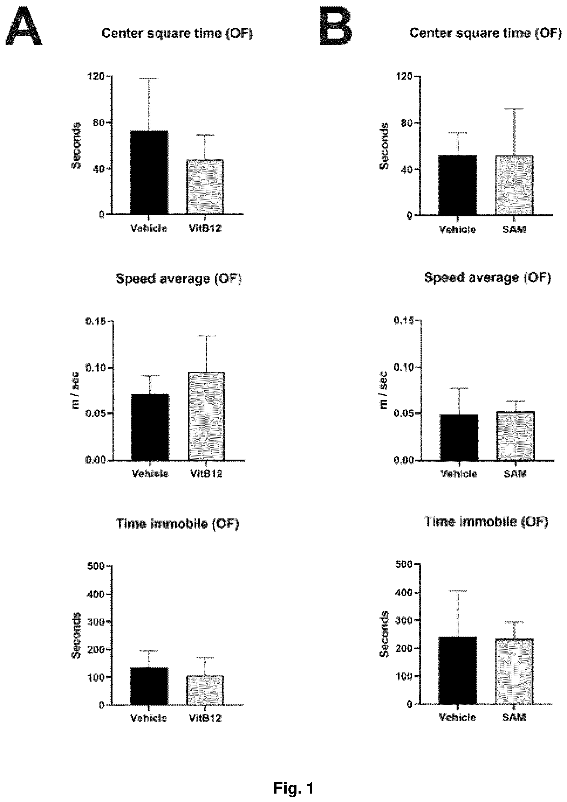

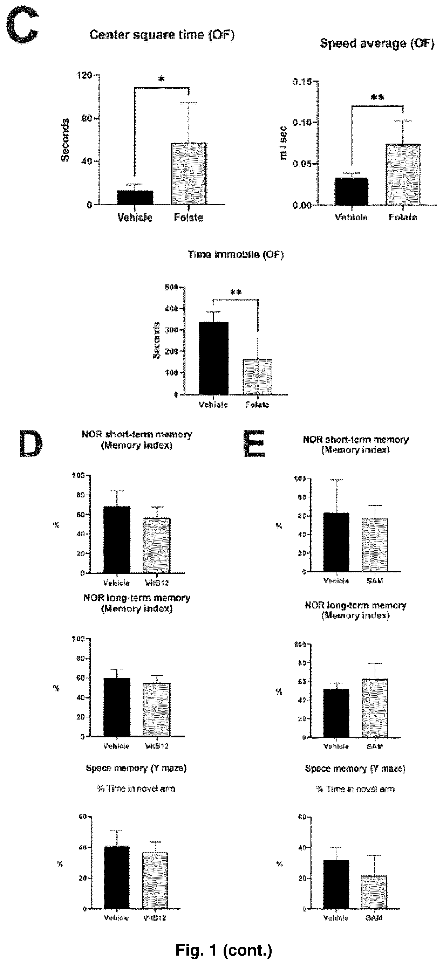

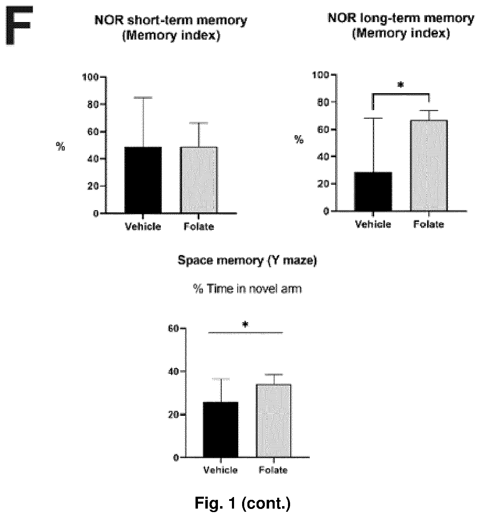

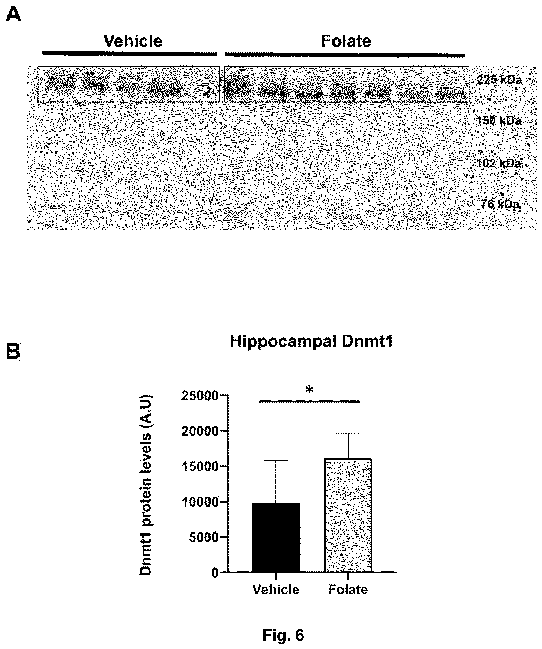

On the other hand, inventors have shown that folate promotes the expression of DNA methyl transferase 1 protein, DNMT1, (Fig. 6). Dnmt1 is involved in the methylation of DNA heterochromatin. It is known in the state of the art that the loss of heterochromatin biomarkers like DNA methylation is associated with age (Villaponteau, The heterochromatin loss model of aging. Exp Gerontol. 1997 Jul-Oct;32(4-5):383-94; Tsurumi A, Li WX. Global heterochromatin loss: a unifying theory of aging? Epigenetics. 2012 Jul;7(7):680-8) and subsequent cognitive impairment. Inventors have also demonstrated that folate displays differential cognitive effects compared to other compounds implicated in one carbon metabolism (Fig. 1), and the results indicate that this may be due to the interaction with the folate receptor α (FRα). In fact, same long-term recognition memory enhancement induced by FRa binding peptide treatment (as described in the Example section, particularly in 2.8. *Cognitive effects of FRa binding peptide* and 3. *Discussion*) as with folate injections, endorse this hypothesis. Given all, it is revealed that FRa binding peptide may promote or partially mediate the activation of DNMT1 and the "re-methylation" of "heterochromatin" DNA, enhancing cognitive functions (Fig. 8), although the action of FR *α* as transcription factor is also important to improve cognition.

Regarding the impact in neuroplasticity markers, FRa peptide displayed similar effects to those exerted by folate (Example section, 2.7. *Cellular rejuvenation effects of FRα binding peptide on hippocampal region*)*.* This "rejuvenation" effects at a cellular level observed in the hippocampus also supports the effect of the peptide of the invention in the cognitive function improvement.

Thus, the invention provides alternative methods and uses, based on mentioned FRa binding peptide, for the prevention and/or treatment of cognitive disorders, as well as for the amelioration of cognitive function, which are described below.

### Therapeutic use and method of the invention

Based on the cognitive function improvement effects displayed by the FRa binding peptide, a therapeutic use and method in the prevention and/or treatment of cognitive disorders are disclosed in the present document.

Thus, a first aspect of the present invention, also referred as the "therapeutic use of the invention", provides a peptide comprising an amino acid sequence with, at least, 75% of identity to amino acid sequence SEQ ID NO: 1, hereinafter the "peptide of the invention", or a composition comprising said peptide (also referred as the "composition of the invention"), for use in treating and/or preventing a cognitive disorder in a subject.

SEQ ID NO: 1 corresponds to the sequence of a FRa binding peptide with the following sequence:
CTVRTSAEC

The peptide of the invention may comprise in its amino acid sequence conservative amino acid substitutions that maintain the ability of the peptide to improve cognitive function in a subject, preferably, an improvement in cognitive capabilities comprising an amelioration of the subject long-term memory. Tests to ascertain whether the peptide of the invention has the ability to improve mentioned cognitive function in a subject are described in the Examples section and along the description.

In a particular embodiment, the peptide of the invention comprises, or consists of, an amino acid sequence having a sequence identity of, at least, 76, 77, 78, 79, 80, 81, 82, 83, 84, 85, 86, 87, 88, 89, 90, 91, 92, 93, 94, 95, 96, 97, 98 or 99% to SEQ ID NO: 1.

In the present invention, "sequence identity" means the degree of similarity between two amino acid sequences obtained by aligning the two sequences. Depending on the number of common residues between the aligned sequences, a degree of identity expressed as a percentage will be obtained. The degree of identity between two amino acid sequences can be determined by conventional methods, e.g. by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI).

In a more particularly embodiment, the peptide of the invention comprises, or consists of, amino acid sequence SEQ ID NO: 1.

In the context of the present invention, a peptide can be defined as a molecule having among 6 to 150 amino acids residues connected of linked by peptide bonds. In a particular embodiment, the peptide of the invention has a length from 9 to 100 amino acids (including the ends), more in particular from 9 to 50 amino acids (including the ends), from 9 to 40 amino acids (including the ends), from 9 to 30 amino acids (including the ends) or from 9 to 20 amino acids (including the ends). In a still more particular embodiment, the peptide of the invention has a length of 9 to 16 amino acids (including the ends). Preferably, the peptide of the invention has a length of 9 amino acids.

Peptides can be obtained by techniques or methods known in the state of the art. Examples of techniques for polypeptide obtention include, without limitation to, chemical or biological synthesis, genetic recombination or expression of polynucleotides coding for the peptide of interest.

Additionally, the carboxyl and amino terminal ends of the peptide of the invention may be protected against proteolysis. For example, the amino-terminal end may be in the form of an acetyl group and/or the carboxyl-terminal end may be in the form of an amide group. It is also possible to carry out internal modifications of the peptides to make them resistant to proteolysis, for example, where at least one peptide bridge - CONH - is modified and replaced by a reduced bond (CH2NH), a retroinverse bond (NHCO), an oxymethylene bond (CH2-O), a thiomethylene bond (CH2-S), a ketomethylene bond (CO-CH2), a hydroxyethylene bond (CHOH-CH2), an (N-N) bond, an E-alkene bond or a -CH=CH- bond. The amino acids of the peptide of the invention can be in D-configuration, which can give rise to peptides resistant to proteolysis. The peptides can also be stabilised by intramolecular cross-linking, for example, by modification of at least two amino acid residues with olefin side chains, preferably, C3-C8 alkenyl chains, preferably, pentel-2-yl chains, followed by cross-linking of the chains as described in the so-called "staple" technology (Walensky et al., 2004, Science 205: 1466-1470). All these peptides chemically modified to resist proteolysis are also covered by the present invention.

Further modifications to the peptide of the invention may comprise covalent binding to a polyethylene glycol (PEG) molecule at its carboxyl-terminal end or to a lysine residue. The half-life of the peptide can also be increased by embedding the peptide in a biodegradable and biocompatible polymeric material to form microspheres that are used as a drug delivery system. Polymers and copolymers are, for example, poly(D,L-lactide-co-glycolic acid) or PLGA. The techniques and procedures of how to manufacture lipid microspheres or nanocapsules for use in drug delivery are widely known to the person skilled in the art.

Furthermore, the peptide of the invention may form part of a composition, hereinafter, the "composition of the invention".

The composition of the invention may be formulated for pharmaceutical administration, i.e., forming part of pharmaceutical products to be administered to the subject by any means of administration. Thus, in a particular embodiment, the composition of the invention is a pharmaceutical composition ("pharmaceutical composition of the invention"). The term "pharmaceutical composition" also encompasses veterinary compositions.

In a particular embodiment, alone or in combination with other particular embodiments, the peptide of the invention is in an effective amount. More preferably, the peptide of the invention is in a therapeutically effective amount.

A "therapeutically effective amount" as used herein includes within its meaning a non-toxic but sufficient amount of a compound, active ingredient, or agent, in the present invention, the peptide of the invention, or composition comprising the same, for use in the embodiments disclosed herein to provide the desired therapeutic/prophylactic effect. Similarly, "an amount effective to", as used in the present document, includes within its meaning a non-toxic but sufficient amount of a compound, active ingredient, or agent, in the present invention, the peptide of the invention, or composition comprising the same to provide the desired effect. The exact amount of the active ingredient disclosed herein required will vary from subject to subject depending on factors such as the species being treated, the age and general condition of the subject, the severity of the condition being treated, the particular agent being administered, the weight of the subject, and the mode of administration and so forth. Thus, it is not possible to specify an exact "effective amount." However, for any given case, an appropriate "effective amount" may be determined by one of ordinary skill in the art using routine methods.

In a particular embodiment, alone or in combination with other particular embodiments, the composition of the invention comprises the peptide of the invention at any suitable concentration. Preferably, the peptide of the invention concentration is between 1 to 5 mg/ml (including the end values of the range), more preferably, between 2.5 mg/ml to 5 mg/ml (including the end values of the range).

In a particular embodiment, alone or in combination with other particular embodiments, the pharmaceutical composition of the invention further comprises a pharmaceutically acceptable carrier and/or excipient.

The term "excipient" refers to a substance that helps the absorption of any components or compounds of the composition of the invention, namely, of peptide of the invention, or stabilizes the components or compounds and/or assists the preparation of the pharmaceutical composition in the sense of giving it consistency or flavours to make it more pleasant. Thus, the excipients may have the function, by way of example but not limited thereto, of binding the components (for example, starches, sugars or cellulose), sweetening, colouring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physical and chemical properties of the pharmaceutical composition and its bioavailability. The "pharmaceutically acceptable" excipient must allow the activity of components or compounds of the pharmaceutical composition, that is, be compatible with the effect exerted by the peptide of the invention.

The "galenic form" or "pharmaceutic form" is the configuration to which the active ingredients and excipients are adapted to provide a pharmaceutical composition or a drug. It is defined by the combination of the form in which the pharmaceutical composition is presented by the manufacturer and the form in which it is administered.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the composition will be adapted to the type of administration used. Thus, the composition may be presented in the form of solutions or any other form of permissible administration and in an effective amount. The composition of the invention can be formulated into solid, semisolid or liquid preparations, such as tablets, capsules, powders, granules, solutions, suppositories, gels or microspheres. In a particular embodiment, the composition of the invention is formulated in liquid form or in solid form.

In another particular embodiment, alone or in combination with other particular embodiments, the solid formulation is selected from the group consisting of tablets, lozenges, sweets, chewable tablets, chewing gums, capsules, sachets, powders, gels, granules, coated particles or coated tablets, tablet, pills, troches, gastro-resistant tablets and capsules and dispersible strips and films.

In another particular embodiment, alone or in combination with other particular embodiments, the liquid formulation is selected from the group consisting of oral solutions, suspensions, droplets, emulsions and syrups.

Likewise, various systems are known that can be used for sustained-release administration of the composition of the invention, including, for example, the encapsulation in liposomes, microbubbles, microparticles or microcapsules and the like. The suitable sustained-release forms as well as materials and methods for their preparation are well known in the state of the art. Thus, the orally administrable form of the composition of the invention is in a sustained-release form further comprising at least one coating or matrix. The sustained release coating or matrix includes, without limitation, natural semisynthetic or synthetic polymers, water-insoluble or modified, waxes, fats, fatty alcohols, fatty acids, natural, semisynthetic or synthetic plasticizers or a combination of two or more of the same. Enteric coatings can be applied using conventional processes known to those skilled in the art.

Such compositions and/or formulations thereof can be administered to an animal, including a mammal and thus a human being, in a variety of forms, including, but not limited to, intracerebral, intrahippocampal, intraperitoneal, intravenous, intramuscular, subcutaneous, intrathecal, intraventricular, intraarticular, oral, enteral, parenteral, intranasal, ocular or topical. A preferred route of administration of the compositions and/or formulations of the peptide of the invention for the prevention or treatment of a cognitive disorder is the intracerebral, intranasal and oral route. In a particular embodiment, the composition of the invention is formulated for oral, intranasal, or intracerebral administration.

As previously mentioned, in the first aspect of the invention, the peptide of the invention, or composition comprising said peptide (also referred in the present document as the "composition of the invention"), is provided for prophylactic and/or therapeutic purposes, particularly, for use in treating and/or preventing a cognitive disorder in a subject.

As used herein, the term "treat", "treating" or "treatment", comprises inhibiting the disease or pathological condition, i.e., stopping its development; relieving the disease or pathological condition, i.e., causing regression of the disease or pathological condition; and/or stabilizing the disease or pathological condition in a subject.

As used herein, the terms "prophylactic treatment," "prevent," or "preventing," refers to the avoidance of the occurrence of a disease or condition in a subject, particularly when the subject has predisposition for the pathological condition, but has not yet been diagnosed and/or treating a subject who does not yet exhibit symptoms of a disease or condition, but who is susceptible to, or otherwise at risk of, a particular disease or condition, whereby the treatment reduces the likelihood that the patient will develop the disease or condition.

In the present invention, the disease or condition is a cognitive disorder. The term ""cognitive disorder" refers to mental health disorders that may primarily affect learning, memory, perception, problem solving and behavioural conditions.

Cognitive disorders may be identified by one or more physical diagnostic tests, including, but not limited to, physical examination by a medical doctor, nurse, physician's assistant, psychologist, counsellor, or other medical professional.

Physical examination may comprises one or more medical imaging evaluations, including, but not limited to x-ray, CT scan, PET scan, MRI, and/or fMRI. Diagnosis and/or identification may also be obtained using established guidelines. Physical examination may also comprises self-examination.

Furthermore, cognitive disorders may also be identified by diagnostic cognitive tests. Examples of diagnostic cognitive tests, include, without limitation to, Modified Mini-Mental State Examination (3MS); Three Word Recall (3WR); 7-Minute Screen (7MS); AB Cognitive Screen (ABCS); Addenbrooke's Cognitive Examination (Revised) (ACER); Abbreviated Mental Test (AMT); Brief Alzheimer Screen (BAS); Brief Cognitive Scale (BCS); Cognitive Abilities Screening Instrument (CASI); Cognitive Assessment Screening Test (CAST); Cognitive Capacity Screening Examination (CCSE); Clock drawing test (CDT); Deterioration Cognitive Observee (DECO); DemTect; Dementia Questionnaire (DQ); General Practitioner Assessment of Cognition (GPCOG); Hopkins Verbal Learning Test (HVLT); Informant Questionnaire on Cognitive Decline in the Elderly (IQCODE); Informant Questionnaire on Cognitive Decline in the Elderly - Short Form (IQCODE-SF); Minnesota Cognitive Acuity Screen (MCAS); Mini-Cog; Memory Impairment Screen (MIS); Mini-Mental State Examination (MMSE); Mont Montpellier Screen (MMS); Neurobehavioral Cognitive Status Examination (NCSE); Rotterdam Version of the Cambridge Cognitive Examination (R-CAMCOG); Rapid Dementia Screening Test (RDST); Short and Sweet Screening Instrument (SASSI); Symptoms of Dementia Screener (SDS); Six Item Screener (SIS); Short Orientation Memory Concentration Test (S-OMC); Short Portable Mental Status Questionnaire (SPMSQ); Short Test of Mental States (STMS); Time and Change (T&C); Telephone Interview of Cognitive Status-Modified (TICS-M); Trail making Test (TMT); Verbal Fluency - Categories (VFC); Modified WORLD Test (WORLD).

Examples of cognitive disorders include, without limitation to, Alzheimer's disease, Huntington's disease, frontotemporal lobar degeneration (FTLD), mild cognitive impairment (MCI), age-related long-term memory loss, Korsakoff syndrome, vascular dementia or Lewy body dementia.

A particular embodiment, alone or in combination with other particular embodiments, provides the peptide of the invention, or the composition comprising said peptide, for use in treating and/or preventing a cognitive disorder in a subject, wherein the cognitive disorder is selected from the list consisting of Alzheimer's disease, Huntington's disease, MCI, age-related long-term memory loss, FTLD, Korsakoff syndrome, vascular dementia, Lewy body dementia, progressive supranuclear palsy and any combination thereof.

In a particular embodiment, alone or in combination with other particular embodiments, the cognitive disorder comprises, or consists of, a long-term memory impairment.

The term "memory", can be defined as the collection of information gained from past learning or experience that is stored in a subject's mind. A piece of information, such as the mental image of an experience, that is stored in the memory. The ability to remember past experiences or learned information, involving advanced mental processes such as learning, retention, recall, and recognition and resulting from chemical changes between neurons in several different areas of the brain, including the hippocampus. Included are (1) declarative learning or memory, which refers to which can be consciously recalled such as facts and knowledge, (2) working memory, which refers to actively holding multiple pieces of transitory information in the mind where they can be manipulated, (3) reference memory, which refers to information gained from previous experience, either recent or remote, (4) recognition memory, which may be defined as the ability to recognize previously encountered events, objects, or people, (5) spatial memory, which may be defined as the maintenance of the information of spatial locations and (6) associative memory, which is the ability to learn and remember the relationship between unrelated items. Each of these has and immediate, short-term, and long-term component. Immediate memory lasts for just a few seconds. Short-term memory stores information that has been minimally processed and is available only for a few minutes, as in remembering a phone number just long enough to use it. Short-term memory is transferred into long-term memory, which can last for more than 24 hours. In the present invention, "long-term memory" refers to memory held for more than 24 hours.

As used herein, "long-term memory impairment" refers to a condition in which a subject does not have a long-term memory as robust as that expected in normal subjects.

Tests or assays to assess long-term memory impairments in subjects are known in the state of the art. Examples of tests/assays to assess long-term memory impairments in rodents include, without limitation to, one or more of Morris Water Maze Test, Barnes Maze Test, Novel Object recognition, Object location task, Y maze test, active place avoidance task or Fear conditioning. Examples of tests/assays to assess long-term memory impairments in human beings include, without limitation to, a Rey Auditory and Verbal Learning Test (RAVLT); Cambridge Neuropsychological Test Automated Battery (CANTAB); a Children's Memory Scale (CMS); a Contextual Memory Test; a Continuous Recognition Memory Test (CMRT); a Denman Neuropsychology Memory Scale; a Fuld Object Memory Evaluation (FOME); a Graham-Kendall Memory for Designs Test; a Guild Memory Test; a Learning and Memory Battery (LAMB); a Memory Assessment Clinic Self-Rating Scale (MAC-S); a Memory Assessment Scales (MAS); a Randt Memory Test; a Recognition Memory Test (RMT); a Rivermead Behavioral Memory Test; a Russell's Version of the Wechsler Memory Scale (RWMS); a Test of Memory and Learning (TOMAL); a Vermont Memory Scale (VMS); a Wechsler Memory Scale; and a Wide Range Assessment of Memory and Learning (WRAML); First-Last Name Association (Youngjohn J.R., et al., Archives of Clinical Neuropsychology 6:287-300 (1991)); Name-Face Association; Wechsler Memory Scale-Revised; (Wechsler, D., Wechsler Memory Scale-Revised Manual, NY, NY, The Psychological Corp. (1987)); California Verbal Learning Test-Second Editi on ( Delis, D.C., et al., The Californian Verbal Learning Test, Second Edition, Adult Version, Manual, San Antonio, TX: The Psychological Corporation (2000)); Facial Recognition (delayed non-matching to sample); Cognitive Drug Research (CDR) Computerized Assessment Battery-Wesnes; Buschke's Selective Reminder Test (Buschke, H., et al., Neurology 24:1019-1025 (1974 )); Telephone Dialing Test; and Brief Visuospatial Memory Test-Revised.

In a particular embodiment, alone or in combination with other particular embodiments, the long-term memory impairment affects to spatial memory and/or recognition memory.

"Spatial memory" can be defined as the maintenance of the information of spatial locations. More particularly, it is related to the storage and retrieval of information within the brain that is needed both to plan a route to a desired location and to remember where an object is located or where an event occurred. Spatial memory is one of the domains of cognition that is often impaired in cognitive disorders such as in Alzheimer's disease, Huntington's disease, MCI, or other dementia-related diseases. Tests or assays to assess spatial memory are known in the art, including without limitation to, one or more of Morris Water Maze Test, Y-maze Test, T maze, Star maze or Barnes maze Test, when the assessment is carried out in rodents, or Corsi-Block-Tapping-Test, Object location test (virtual environment), when the assessment is to be carried out in humans.

"Recognition memory" can be defined as the ability to identify as the ability to recognize previously encountered events, objects, or people. Recognition memory is one of the domains of cognition that is often impaired in cognitive disorders such as in Alzheimer's disease or Korsakoff syndrome. Tests or assays to assess recognition memory are known in the art, including without limitation to, one or more of novel object recognition test (NOR), object-context recognition test or Y-maze Test for rodents, and recognition memory test (RMT) such as Recognition Memory for Words (RMW) or Recognition Memory for Faces (RMF), and visual recognition memory test for humans.

In a particular embodiment, when the cognitive disorder comprises a long-term memory impairment, the cognitive disorder is selected from the list consisting of Alzheimer's disease, Huntington's disease, MCI, age-related long-term memory loss, Korsakoff syndrome, FTLD, vascular dementia, Lewy body dementia, progressive supranuclear palsy, and any combination thereof.

Within the context of the present invention, the term "subject" relates to any animal from any species. In a particular embodiment, the subject is a mammal, preferably a primate or a rodent, such as *Mus musculus.*

Inventors have assessed cognitive function in mice. As a person skilled in the art knows, mouse models are extensively used to model and evaluate cognitive impairments and/or changes in the cognitive function, for instance, in the searching and development of compounds with therapeutic effects in human beings, as well as compounds able to ameliorates cognitive function also in human beings. Thus, in a more particular embodiment, the subject is a human.

In addition, this group of mice were aged approximately 20 months, which is considered to be an elderly age. Thus, in another particular embodiment, alone or in combination with other particular embodiments, the subject is an elderly subject.

In a particular embodiment, alone or in combination with other particular embodiments, the subject is experiencing normal aging cognitive changes.

In a particular embodiment, alone or in combination with any other particular embodiments, the subject is over 40 years old, preferably over 50 years, more preferably over 60 years, even more preferably over 70 years old.

In a particular embodiment, alone or in combination with other particular embodiments, the subject is suffering a cognitive disorder. The term "cognitive disorder" has been previously described, and its definition, as well as its preferred embodiments, apply equally herein.

Other aspect of the present invention relates to a method for treating and/or preventing a cognitive disorder in a subject, hereinafter referred as the "therapeutic method of the invention", which comprises administering the peptide of the invention, or the composition of the invention, to the subject.

Terms used to define the therapeutic method of the invention have been previously described, and its definitions, as well as their preferred embodiments, apply to the present aspects of the invention.

Other aspect of the present invention provides the use of the peptide of the invention or the composition of the invention, for the manufacture of a medicament, wherein the medicament is for treating and/or preventing a cognitive disorder in a subject.

Terms, definitions, as well as their preferred embodiments, have been previously described, and apply equally to the present aspects of the invention.

### Non-therapeutic method and use of the peptide of the invention or the composition comprising said peptide

As previously described, the peptide of the invention, or the composition comprising said peptide is able to improve cognitive function in a subject.

Thus, in other aspect, the invention provides a non-therapeutic use of the peptide of the invention, or a composition comprising said peptide, the composition of the invention, for improving cognitive function in a subject.

The terms "cognition", "cognitive function" and "cognitive performance" are used herein interchangeably and are related to any mental process or state that involves but is not limited to learning, memory, creation of imagery, thinking, awareness, reasoning, spatial ability, speech and language skills, language acquisition and capacity for judgment attention. Cognition is formed in multiple areas of the brain such as hippocampus, cortex and other brain structures. It is assumed that long term memories are stored at least in part in the cortex and it is known that sensory information is acquired, consolidated and retrieved by a specific cortical structure, the gustatory cortex, which resides within the insular cortex. Another region that has been shown to be responsible for memory formation and long-term storage is hippocampus. The phrase "improving cognitive function", refers to the amelioration of physiologic activity of the brain or the enhancement of mental processes or states, that may include, without limitation to learning, memory, creation of imagery, thinking, awareness, reasoning, spatial ability, speech and language skills, language acquisition and capacity for judgment attention.

Cognitive function may be measured by known methods, for example and without limitation, by the clinical global impression of change scale (CIBIC-plus scale); the Mini Mental State Exam (MMSE); the Neuropsychiatric Inventory (NPI); the Clinical Dementia Rating Scale (CDR); the Cambridge Neuropsychological Test Automated Battery (CANTAB) or the Sandoz Clinical Assessment-Geriatric (SCAG). Cognitive function may also be measured indirectly using imaging techniques such as Positron Emission Tomography (PET), functional magnetic resonance imaging (fMRI), Single Photon Emission Computed Tomography (SPECT), or any other imaging technique that allows one to measure brain function.

In a particular embodiment, the improvement of the cognitive function comprises an amelioration of the subject long-term memory.

Test or assays to assess long-term memory in a subject are known in the state of the art. Examples of tests/assays to assess long-term memory amelioration in rodents include without limitation to, one or more of Morris Water Maze Test, Barnes Maze Test, Novel Object recognition, Object location task, Y maze test, active place avoidance task or Fear conditioning. Examples of tests/assays to assess long-term memory amelioration in human beings include, without limitation to, a Rey Auditory and Verbal Learning Test (RAVLT); Cambridge Neuropsychological Test Automated Battery (CANTAB); a Children's Memory Scale (CMS); a Contextual Memory Test; a Continuous Recognition Memory Test (CMRT); a Denman Neuropsychology Memory Scale; a Fuld Object Memory Evaluation (FOME); a Graham-Kendall Memory for Designs Test; a Guild Memory Test; a Learning and Memory Battery (LAMB); a Memory Assessment Clinic Self-Rating Scale (MAC-S); a Memory Assessment Scales (MAS); a Randt Memory Test; a Recognition Memory Test (RMT); a Rivermead Behavioral Memory Test; a Russell's Version of the Wechsler Memory Scale (RWMS); a Test of Memory and Learning (TOMAL); a Vermont Memory Scale (VMS); a Wechsler Memory Scale; and a Wide Range Assessment of Memory and Learning (WRAML); First-Last Name Association (Youngjohn J.R., et al., Archives of Clinical Neuropsychology 6:287-300 (1991)); Name-Face Association; Wechsler Memory Scale-Revised; ( Wechsler, D., Wechsler Memory Scale-Revised Manual, NY, NY, The Psychological Corp. (1987)); California Verbal Learning Test-Second Editi on ( Delis, D.C., et al., The Californian Verbal Learning Test, Second Edition, Adult Version, Manual, San Antonio, TX: The Psychological Corporation (2000)); Facial Recognition (delayed non-matching to sample); Cognitive Drug Research (CDR) Computerized Assessment Battery-Wesnes; Buschke's Selective Reminder Test (Buschke, H., et al., Neurology 24:1019-1025 (1974 )); Telephone Dialing Test; and Brief Visuospatial Memory Test-Revised.

As previously mentioned, in the present invention, "long-term memory " refers to memory held for more than 24 hours.

In a particular embodiment, alone or in combination with other particular embodiments, the cognitive function is selected from the list consisting of learning, memory, preferably wherein memory is at least one of spatial memory, recognition memory, visuospatial and relational working memory, complex attention, executive functioning, and any combination thereof.

In a particular embodiment, alone or in combination with other particular embodiments, the long-term memory amelioration comprises improving spatial memory and/or recognition memory.

In another particular embodiment, alone or in combination with other particular embodiments, the improvement of the cognitive function is an amelioration of the long-term memory.

In a particular embodiment, the subject is a mammal, preferably a primate or a rodent, such as *Mus musculus.* In a more particular embodiment, the subject is a human.

In another particular embodiment, alone or in combination with other particular embodiments, the subject is over 40 years old, preferably over 50 years, more preferably over 60 years, even more preferably over 70 years old.

In another particular embodiment, alone or in combination with other particular embodiments, the subject is an elderly subject.

In another particular embodiment, alone or in combination with other particular embodiments, the subject is a healthy subject.

In another particular embodiment, alone or in combination with other particular embodiment, the subject is experiencing normal aging cognitive changes.

Some terms used to define the non-therapeutic use of the invention have been previously described, and its definitions, as well as their preferred embodiments, apply to the present aspect of the invention.

Other aspect of the invention refers to a non-therapeutic method for improving cognitive function in a subject, hereinafter the "non-therapeutic method of the invention", which comprises administering the peptide of the invention, or the composition of the invention, to a subject.

Terms used to define the non-therapeutic method of the invention have been previously described, and its definitions, as well as their preferred embodiments, apply to the present aspect of the invention.

### DESCRIPTION OF THE DRAWINGS

**Fig. 1****. Effect of vitamin B12, SAM and Folate on behavioral changes.** Unique dose of hippocampal infusion of Folate has revealed behavioral changes in old wild type C57BJ mice comparing with vehicle solution application, but not for Vitamin B12 or S-adenosylmethionine metabolite (SAM) infusion. (A,B,C) Histograms show some of the main data obtained from open field test: time spent by mice in the center square of the box during the test, speed average of mice movement and time in which mice remained immobile during the test. Folate-infused mice showed an improvement in anxiety-depression-like behavior (indicated by the time spent in the center square) as well as in general motor activity (illustrated by the rest of histograms). (D,E,F) Histograms show results from two different memory performance tests: novel object recognition test (NOR) for short and long-term recognition memory, and Y maze test for spatial memory. Significant differences were found in Folate-injected mice in comparison with vehicle group, but not were found between Vitamin B12 or SAM-injected mice groups regarding vehicle-injected mice in any memory test. Black bars represent mean± SD of vehicle experimental mice group and grey bar represent mean± SD of Vitamin B12/SAM/Folate-infused mice group. All data were analyzed by Student's t-tests. *p<0.05 , **p<0.01

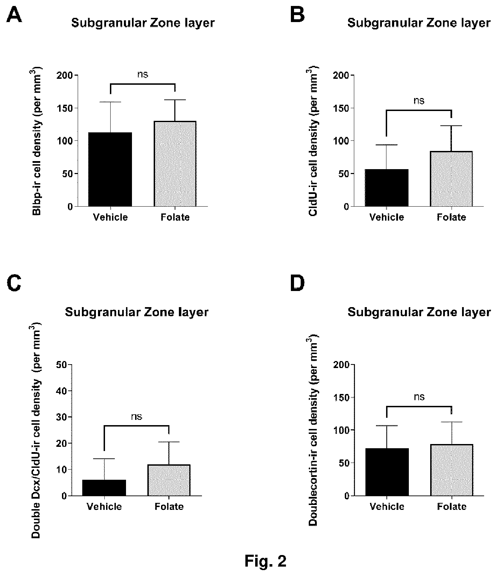

**Fig. 2****. Effect of folate on the expression of adult neurogenesis markers. (A)** Histogram showing density of Blbp-ir cells per mm³ obtained in the subgranular zone (SGZ) of dentate gyrus. Unique-dose treatment with folate compound have not resulted in statistically significant changes in early neural precursors in the SGZ. **(B)** Histogram showing density of CldU-ir cells per mm³ obtained in the subgranular zone (SGZ). Unique-dose treatment with folate compound have not resulted in statistically significant changes in the generation of new cells in the SGZ. **(C)** Histogram showing density of double CldU/doublecortin-ir cells per mm³ obtained in the subgranular zone layer (SGZ). Unique-dose treatment with folate compound have not resulted in statistically significant changes in the density of new 2-weeks-old neurons in the SGZ. **(D)** Histogram showing density of doublecortin-ir cells per mm³ obtained in the subgranular zone layer (SGZ). Unique-dose treatment with folate compound have not resulted in statistically significant changes in the density of young neurons in the SGZ. (Mean±SD; ns: not significant differences; Student's t test). Scale bar, 100 µm.

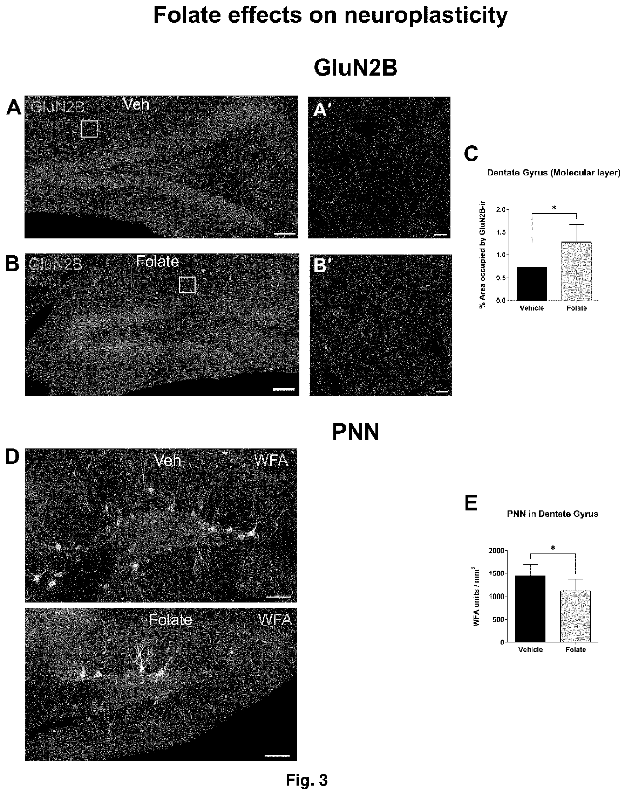

**Fig. 3****. Rejuvenation effects of folate on plasticity markers. (A)** Effects on the presence of GluN2B in mature hippocampal neurons. On the left are shown representative stitched confocal images of GluN2B distribution in dentate gyrus (DG) of old wild-type mice treated with vehicle or folate. On the right, it is shown the levels of GluN2B expression obtained determining the percentage of area occupied by the marker in the molecular layer of DG. Treatment with folate compound induced a significant increase of GluN2B expression in the DG (Mean±SD; *p<0.05, Student's t test). Scale bar, 100 µm. **(B)** Effect of folic acid on extracellular matrix. On the left are represented stitched confocal images (20x) of immunofluorescence staining for WFA as a marker of perineuronal nets (PNNs) in the dentate gyrus from vehicle-injected and folate-injected mice. Inset shows also Dapi signal to identify DG structure. On the right it is shown an histogram displaying the density of CldU-ir cells per mm³ obtained in the subgranular zone (SGZ). Unique-dose treatment with folate compound have resulted in statistically significant changes in the presence of PNN units density at dentate gyrus. (Mean±SD; *p<0.05, Student's t test). Scale bar, 100 µm.

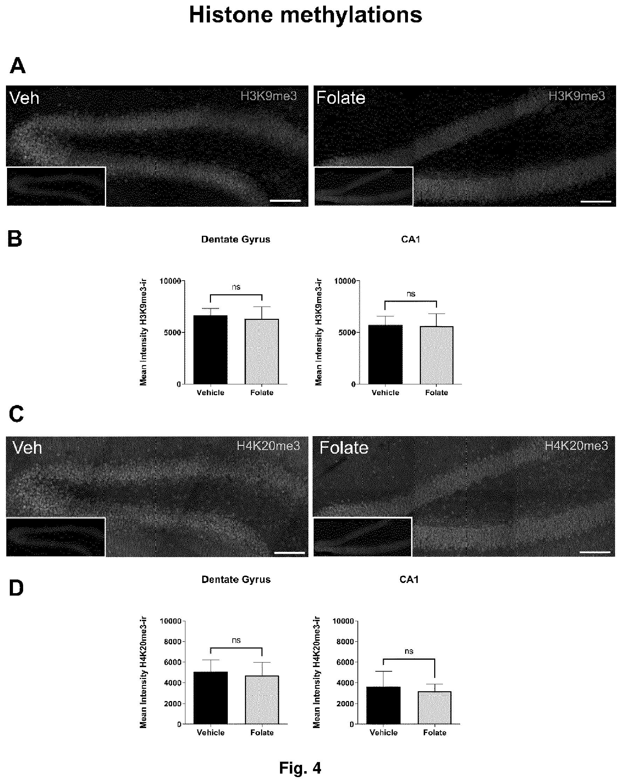

**Fig. 4****. Levels of H3K9me3 and H4K20me3. Effect of folate. (A)** Representative stitched confocal images of H3K9me3 marker in the dentate gyrus. Inset shows also Dapi signal to identify DG structure. **(B)** Histogram showing mean intensity of histone methylation signal. The effect of the absence (black) or presence (grey) of folic acid is shown. **(C)** Representative stitched confocal images of H4K20me3 marker in the dentate gyrus. Inset shows also Dapi signal to identify DG structure. **(D)** Histogram showing mean intensity of histone methylation signal. The effect of the absence (black) or presence (grey) of folic acid is shown. (Mean±SD; ns: not significant differences; Student's t test). Scale bar, 100 µm.

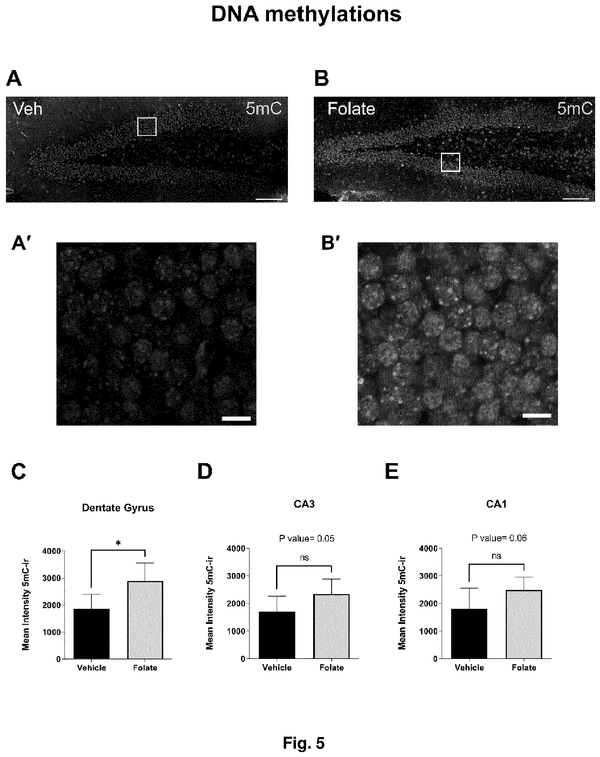

**Fig. 5****. Effect of folate on DNA methylation of hippocampal neurons.** (A,B) Representative stitched confocal images of 5-Methylcytosine (5mC) marker in the dentate gyrus from vehicle-injected and folate-injected mice. A' and B' are the corresponding higher magnifications from the granular cell layer of A and B (white squares). In A' and B' can be visualized heterochromatin clusters of 5mC-immunoreactivity. **(C,D,E)** Histogram showing mean intensity from 5mC signal of the granular cell layer of DG, and from pyramidal cell layer of CA3 and CA1 hippocampal regions. The effect of the absence (black) or presence (grey) of folic acid is shown. (Mean±SD; *p<0.05, Student's t test; ns: not significant differences). Scale bar, 100 µm.
**Fig. 6****. Effect of folate on DNA methyl transferase 1 protein levels.** (A) Detection of Dnmt1 in hippocampal samples by Western blotting. (B) Histogram showing densitometry (arbitrary units) of western blotting. The effect of the absence (black) or presence (grey) of folic acid is shown. (Mean±SD; *p<0.05, unpaired non-parametrical Kolmogorov-Smirnov test).
**Fig. 7****. Effect of FRα activator peptide, with SEQ ID NO: 1 on recognition memory performance.** Histograms show results from two different memory performance tests: novel object recognition (NOR) for short and long-term recognition memory. Peptide-infused mice showed a significant improvement in memory retention after 5 days since first training. FRa peptide, with SEQ ID NO: 1, infusion have not showed significant effect in short term recognition memory. Black bars represent mean± SD of vehicle experimental mice group and grey bar represent mean± SD of peptide-infused mice group. All data were analyzed by Student's t-tests. *p<0.05
**Fig. 8** **DNA methylation recovery in aged cells.** Left - Heterochromatin loss aging model as described in some documents belonging to the state of the art, indicating the loss of heterochromatin biomarkers like DNA methylation (in heterochromatin regions) with age. Right - Proposed model/working hypothesis: folate or FRa binding peptide may facilitate the transport of FRa to the cell nucleus, where FRa is acting like a transcription factor (Boshnjaku et al., Nuclear localization of folate receptor alpha: a new role as a transcription factor. Sci Rep 2: 980 (2012)) promoting the expression of genes like DNMT1. It facilitates the methylation of heterochromatin DNA, previously demethylated, going back to the state found in younger cells. This "rejuvenation" in neurons may facilitate cognitive improvement.

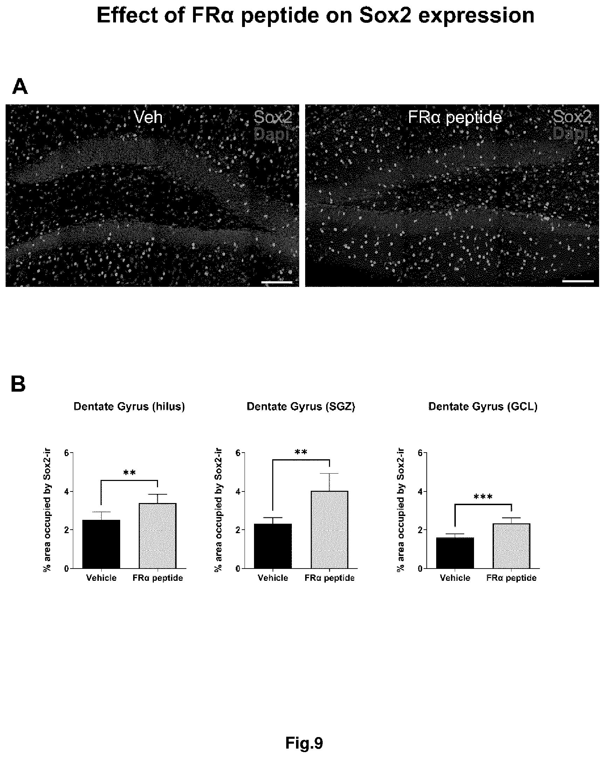

**Fig. 9** **Effect of folate receptor alpha (FRα) peptide, with SEQ ID NO: 1, on Yamanaka factor Sox2 expression.** (A) Representative stitched confocal images of Sox2 expression in the dentate gyrus from vehicle-injected and FRα-injected mice. (B) Unique-dose treatment with FRa compound have resulted in statistically significant changes in the presence of Sox2 immunoreactivity expression at hilus region, granular cell layer (GCL) and in subgranular zone (SGZ) of the dentate gyrus. (Mean±SD; *p<0.05, **p<0.01; Student's t test). Scale bar, 100 µm.

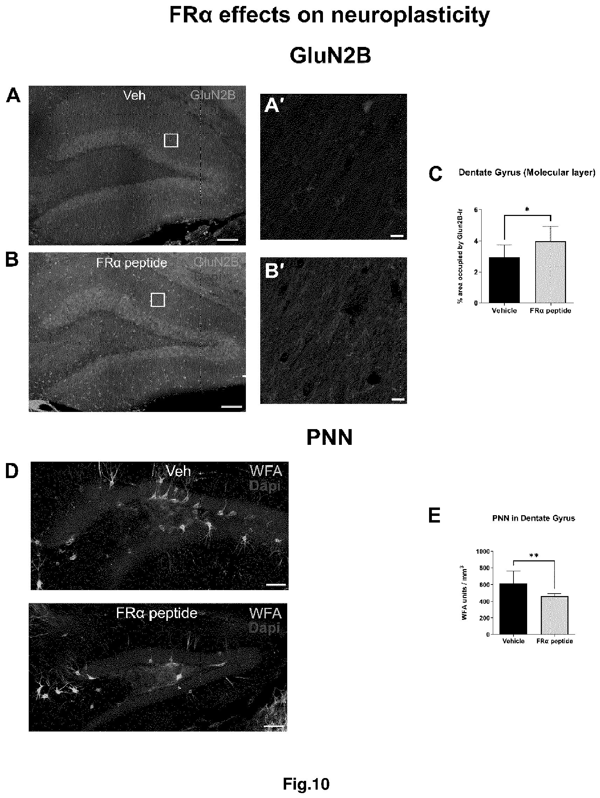

**Fig. 10** **Effects of folate receptor alpha (FRα) binding peptide, with SEQ ID NO: 1, on GIuN2B expression and extracellular matrix organization.** Effects on the presence of NMDA receptor subunit GluN2B in mature hippocampal neurons. (A, B) Representative stitched confocal images of GluN2B distribution in dentate gyrus (DG) of old wild-type mice treated with vehicle or FRa peptide. A' and B' are the corresponding higher magnifications from the molecular cell layer of A and B (white squares). (C) Histogram showing the percentage of area occupied by GluN2B signal in the molecular layer of DG. The effect of the absence (black) or presence (grey) of FRa is shown. Treatment with FRa compound induced a significant increase of GluN2B expression in the DG (Mean±SD; *p<0.05, Student's t test). (D) Representative stitched confocal images of Wisteria floribunda agglutinin (WFA, in green), as a marker of perineuronal nets (PNN) in the dentate gyrus from vehicle-injected and FRa -injected mice. (E) Unique-dose treatment with FRa compound have resulted in statistically significant changes in the presence of PNN density at dentate gyrus. (Mean±SD; *p<0.05, **p<0.01; Student's t test). Scale bar, 100 µm (A, B and D) and 10 µm (A' and B').

### Examples

### 1. Materials and methods

### 1.1. Animals

A total of 43 C57BL/6 male and female mice were bred in the animal facility at the Centro de Biologia Molecular Severe Ochoa, housed in a specific pathogen-free colony facility under standard laboratory conditions following European Community Guidelines (directive 86/609/EEC), and handled in accordance with European and local animal care protocols (PROEX 62/14 and 291/15). They were housed 4-5 per cage with food and water available ad libitum, and maintained in a temperature controlled environment on a 12/12-h light/dark cycle with light onset at 8 a.m.

### 1.2. Experimental design

Different experimental group of old mice (~20 months of age) were randomly injected on the hilus region of both brain hemispheres with vehicle (PBS or destillated water) or with one of four different molecules: Vitamin B12 (50 mg/ml; Vet one, ref: 510218), S-(5'-Adenosyl)-L-methionine chloride (0,25 mg/ml; Sigma, ref: A7007), folic acid (0,25 mg/ml; Sigma, ref: F7876-10G) or synthetic FRa binding peptide, peptide having amino acid sequence SEQ ID NO: 1, (2,5 mg/ml and 5 mg/ml, diluted in PBS; Abyntec). The pH of Vitamin B12, methionine or folic acid were adjusted to ~7 to avoid negative effects due to overly acidic medium. One week after intracerebral injections, mice were assessed with behavioural (open field trial) and memory tests (novel object recognition) to study potential functional effects of different metabolites on central nervous system. Two weeks after surgeries mice were finally perfused just after finalized Y maze test performing.

### 1.3. Stereotaxic surgery and intrahippocampal microinjection.

After anesthesia with isofluorane, mice were secured in a stereotaxic frame (Kopf). Holes were drilled bilaterally in the skull at the injection sites (one per hemisphere) by microdrill with 0.5 mm drill bit. Stereotaxic coordinates used for intrahippocampal injections were as follows (from bregma): anterior-posterior 2.2, lateral 1.4, dorsoventral 2.0. A 33-gauge needle Hamilton syringe coupled to a syring pump controller mounted to the stereotaxic frame was used to inject 2µl of vehicle or metabolites at each site. Injections occurred at a rate of 0.25 µl/min, after which the needle was left in place for an additional 3 min, stopping another 3 minute in the halfway. After injections were completed, the skin was sutured and the animals were allowed to recover for 1 h on a heating pad before returning to the home cage. Mice remained in the home cage for an additional 1 week before the start of behavioral testing, being sacrificed 2 weeks after surgery.

### 1.4. CIdU injection protocol

Mice received three injections each 2 hours (200 ul/injection, in alternative sides of body) of 5-Chloro-2'-deoxy-Uridine (CldU, Sigma reference C6891; i.p. 42.75 mg/Kg bw) one week after cranial surgery and microinjections of solutions. In this way we were able to follow the effects of treatment in the proliferation of new cells at the subgranular zone, essential zone for neurogenesis process.

### 1.5. Sacrifice and Tissue Processing

Mice were anesthetized with an intraperitoneal pentobarbital injection (Dolethal, 60 mg/kg body weight) and transcardially perfused with saline. Brains were separated into two hemispheres. One hemisphere was removed and fixed in 4% paraformaldehyde in 0.1 M phosphate buffer (PB; pH 7.4) overnight at 4ºC. The next day, it was washed three times with 0.1 M PB and cut along the sagittal plane using a vibratome (Leica VT2100S). Serial parasagittal sections (50 mm thick) were cryoprotected in 30% sucrose solution in PB and stored in ethylene glycol/glycerol at 20ºC until they were analyzed. Regarding the other hemisphere, the hippocampus was rapidly dissected on ice and frozen in liquid nitrogen for western blot.

### 1.6. Immunofluorescence techniques

For immunofluorescence experiments, free floating serial sections (50-µm thick) were first rinsed in PB and then preincubated for 2 h in PB with 0.25% Triton-X100 and 3% normal serum of the species in which the secondary antibodies were raised (Normal Goat Serum / Normal Donkey Serum, Invitrogen, Thermo Scientific). For the study of proliferation and neurogenesis serial sections were previously pre-treated in 2M HCl for 15 min at 30ºC. Subsequently, brain sections were incubated for 24 h at 4 °C in the same preincubation stock solution containing the following primary antibodies in different combinations: ab6326 (Abcam) for CldU; AB2253 (Sigma Aldrich) for Doublecortin; ab8898 (Abcam) for Histone H3 (tri methyl K9); ab78517 (abcam) for Histone H4 (di methyl K20, tri methyl K20); p1516-1480 (Phosphosolution) for NMDA NR2B Subunit; ab32423 (Abcam) for brain lipid-binding protein (Blbp), 33D3 (epigentek) for 5-methylcytosine (5mC) and L1516 (Merck) for Lectin from Wisteria floribunda (WFA).

After rinsing in PB, the sections were incubated for 2 h at room temperature in the appropriate combinations of Alexa 488-594-647-conjugated donkey/goat antimouse/rabbit/guinea pig or rat secondary antibodies (1:500; Molecular Probes, Eugene, OR, USA). Sections were also stained with a nuclear stain DAPI (4,6-diamidino-2-phenylindole; Sigma, St. Louis, MO, U.S.A.).

From the whole dentate gyrus of hippocampus, we obtained stitched image stacks recorded at 1 µm intervals through separate channels with a 20x lens for neurogenesis and extracellular matrix analysis and at 0.6 µm intervals through separate channels with a 40x lens for DNA and histones methylations analysis (Nikon A1R confocal microscope, NA 0.75, refraction index 1, image resolution: 1024 × 1024 pixels). Adobe Photoshop (CS4) software was used to compose figures.

### 1.7. Immunohistochemistry Quantifications

Quantifications for density of DCX/Blbp/CIdU-immunoreactive positive cells were performed by counting in four different consecutive medial brain sections the number of cells localized in each whole DG (distinguishing between SGZ and GCL) and the adjacent hilus region. For perineuronal nets of extracellular matrix, positive units labelled with WFA were counted in the dentate gyrus (including granular cell layer and hilus) from three different consecutive medial brain sections. In all cases the sections were always those closer to the injection area and then localized at the same positions. For determination of the density of different cells, the DG was traced on the DAPI channel of the z projection of each confocal stack of images and by using the freehand drawing tool in Fiji, the area of this structure was measured. This area was multiplied by the stack thickness to calculate the reference volume. The number of positive cells was divided by the reference volume, and the density (number of cells/mm³) of cells was calculated.

For 5mC, H3K9me3 and H4K20me3 antibodies, acquisition confocal settings (laser intensity, gain, pinhole) were kept constant for all images, which were captured in the same confocal session. Using DAPI channel the whole GCL area was previously selected as a ROI. An invariant subtract background and threshold was then set in Fiji for every stack projection. The area above the threshold was then measured in Fiji and the mean fluorescence in the ROI was calculated and compared between vehicle and folate experimental groups.

For GluN2B antibodies, the quantification was carried out with the imaged program by measuring the percentage of area occupied by the receptor immunoreactivity in the molecular layer of dentate gyrus. An invariant subtract background and threshold was also set in Fiji for every stack projection.

### 1.8. Behavioural tests

Both open field (OF) and novel object recognition (NOR) tests were performed as described previously (Rodríguez-Matellán A, et al., In Vivo Reprogramming Ameliorates Aging Features in Dentate Gyrus Cells and Improves Memory in Mice. Stem Cell Reports. 2020 Nov 10;15(5):1056-1066. ). Locomotor activity as well as anxiety and depression-like behaviours in old mice were evaluated using OF. This test was used as habituation of NOR test. In brief, on the first day, the mice were placed individually for 10 min in a 45x45-cm plastic box with vertical opaque walls. Each session was recorded and different data like distance, speed, immobile time and time on center square of the box were analysed with Anymaze software. On the second day, mice were placed in the same box for 5 min, allowing them to explore two identical objects: A and B (two black rooks). Both objects were placed on the long axis of the cage, each 13 cm from the cage end. After each exposure, the objects and the cage were wiped with 70% ethanol to eliminate odors which potentially could condition successive mice behaviour. Two hours after the familiarization trial, each mouse was released into the box with the same object previously used (object A) and a new one (object C, a tower of coloured plastic pieces), instead of the object B (short-term memory test). The position of object C was the same as that of object B in the familiarization trial. The mice were given 5 min to explore the box. Five days later, the mice were released again into the box, with object A in the same position and another new object (object D, a falcon tube filled with wood chips) in the same position of the object C in the previous test. Again, animals were allowed 5 min to explore the box (Long-term memory test). Animals were considered to show recognition when their head was <2 cm from the object.

The memory index (Ml) was used to measure recognition memory performance. It was defined as the ratio of time spent exploring the new object (tC or tD) to the time spent exploring both objects (tA+tC or tA+tD) (MI = [tC/(tA + tC)] X100 and [tD/(tA + tD)] X 100). Anymaze software was used to calculate exploring time of mice in different objects.

To assess spatial memory, an Y maze test based on published protocols with modifications (Kraeuter AK, et al. The Y-Maze for Assessment of Spatial Working and Reference Memory in Mice. Methods Mol Biol. 2019;1916:105-111. ) was performed. This test is based on the innate curiosity of rodents to explore novel areas. Firstly, mice were placed into one of the arms of a Y-maze black apparatus, which is made up of three plastic arms forming a "Y" shape. Mice were then allowed to explore the maze for 10 minutes, with one of the arms closed (training trial). After one-hour interval, mice were returned to the Y maze into the same arm (start arm) and were allowed to explore all three arms of the maze for 5 minutes. The number of entries into and the time spent in each arm, were registered from video recordings and analysed by AnyMaze software. We have compared the percentage of time spent in the "novel" arm during the whole trial as a spatial memory performance measure.

### 1.9. Synthetic synthesis of FRα binding peptide

With the aim of mimic cognitive results produced by folate intrahippocampal injection but activating only FR alpha receptor, we used a synthetic FRa binding peptide (purity of 90%) with the following sequence: CTVRTSAEC (SEQ ID NO: 1). The synthesis of this peptide was performed by Abyntek company (Vizcaya, Spain).

### 1.10. Western Blot

Hippocampal extracts for Western Blot analysis were prepared in homogenization buffer consisting of 50 mM pH 7.4 Tris-HCl, 1% NP-40, 150 mM NaCl, 1 mM EDTA, 0.25% sodium deoxycholate, phosphatase inhibitors (1 mM NaF, 1 mM Na3VO4 and 1 µM okadaic acid) and protease inhibitor cocktail (Complete, Roche). Samples were homogenized at 4 °C in homogenization buffer, and protein content was determined by Bradford Protein Assay (Bio-Rad#500-0006). Total protein-25 µg for the analysis of hippocampal extracts-was electrophoresed on 6% SDS-PAGE gel due to the high molecular weight of the selected protein (Dnmt1, 180kDa), and transferred to a nitrocellulose membrane (Schleicher and Schuell). To avoid non-specific background, membranes were then blocked in PBS 0.1% Tween-20 supplemented with 5% non-fat dry milk and incubated at 4 °C overnight with the following primary antibodies: rabbit anti-Dnmt1 (Abcam, ab87654, 1:500). Secondary antibodies goat anti-rabbit (Dako, 1:1000) followed by ECL detection reagents (Amersham), were used for immunodetection.

### 1.11. Statistical Analysis for immunohistochemical and behaviour assessments

The data are presented as mean values ± SD. Statistical analyses of data were performed using GraphPad Prism 8. For comparisons between two experimental groups (injected with vehicle and with Folate or Activator peptide), an unpaired Student's t test was carried out. A 95% confidence interval was applied for statistical comparisons.

### 2. Results

### 2.1. Partial improvement in the object recognition and Y maze test in mice treated with folate but not with S-Adenosyl-Methionine (SAM) or vitamin B12

Vitamin B12, SAM and folate were intracranially injected at the dorsal hippocampal hilar region in old mice (~20 months) and the effects of those single injections on behavioral tests were analyzed one week after cranial surgeries, once mice were recovered (see Methods). In the first round of surgeries 12 mice were assessed for behavioral changes due to vitamin B12 or vehicle infusion. Open field test has not revealed any alteration in locomotor activity (distance travelled, speed and immobile time) or in anxiety-depression like behavior (central square time) as a consequence of vitamin B12 injection (Fig.1A). Cognitive tests (Fig. 1D), focusing on recognition memory at short (2 hours after training trial) and long-term (5 days after training trial), and in spatial memory, have not revealed significant differences between vehicle and vitamin B12 effects. Further on, we screened another metabolite such as SAM in another group of 9 old mice. Results of behavioral performance shown an absence of effects due to SAM injection in both types tests: open field (Fig.1B and 1E) cognitive tests.

However, when the effect of folate was tested on a new group of 12 old mice (Fig. 1 C,F), differences in both types of tests (open field and cognition) were found. Folate-injected mice shown an improvement in locomotor activity and in anxiety and depression-like behavior regarding vehicle-injected mice. Fig. 1F shows differences in the Y maze test and in the object recognition test when long-term memory (5 days) was assessed.

This result in the presence of folate is similar to that reported for old mice expressing Yamanaka Factor or YF (Rodríguez-Matellán A, et al., In Vivo Reprogramming Ameliorates Aging Features in Dentate Gyrus Cells and Improves Memory in Mice. Stem Cell Reports. 2020 Nov 10;15(5):1056-1066). The presence of folate improves the long-term memory (5 days) post familiarization with the novel object, but not significant differences were found for short-time (2 hours) memory (Fig.1F). In addition, an improvement on spatial memory was observed in folate treated mice.

The finding of behavioural effects on old mice due to folate injection, lead us to examine more in detail feasible cellular effects of folate on these mice.

### 2.2. Effect of folate in dentate gyrus adult neurogenesis

As in our previous work (Rodríguez-Matellán A, et al., In Vivo Reprogramming Ameliorates Aging Features in Dentate Gyrus Cells and Improves Memory in Mice. Stem Cell Reports. 2020 Nov 10;15(5):1056-1066. ), we have tested the possible effect of folate in the presence of neuronal precursors cells, studying changes on brain lipid-binding protein (blbp) or doublecortin-immunoreactive (ir) cell densities. To label dividing cells, mice were injected intraperitoneally with CldU one week before perfusion, allowing the study of new-born one-week-old cells in subgranular zone (SGZ). Our results have found that a unique dose of folate hippocampal injection only led to a subtle but non-significant tendency of increase in early neuronal precursor like blbp-ir cells (Fig.2A), in new born CIdU-ir cells (Fig.2B) and in one-week-old neurons (double Dcx/CldU-ir cells) (Fig.2C) in subgranular zone. Furthermore, we have neither found differences in doublecortin-ir cell density (Fig.2D) due to folate injection. Indeed, in our previous work, a main Yamanaka factor (YF) dependent change was found only in old born compared to new born neurons present in the hippocampal region. Thus, we subsequently tested the effect of folate in old born cells present at dentate gyrus, looking at specific markers.

### 2.3. Potential rejuvenating effects of folate in hippocampus

### Effect of folate in GluN2B levels:

We previously reported (Rodríguez-Matellán A, et al., In Vivo Reprogramming Ameliorates Aging Features in Dentate Gyrus Cells and Improves Memory in Mice. Stem Cell Reports. 2020 Nov 10;15(5):1056-1066 ) that the presence of YF induced an increase in the expression of GluN2B in the molecular layer of the dentate gyrus. This is one of the subunit of N-methyl-D aspartic acid (NMDA) receptor, which is mainly present in young animals and that may be related to a higher cognitive capacities (Paoletti X, et al., Progression-free survival as a surrogate for overall survival in advanced/recurrent gastric cancer trials: a meta-analysis. J Natl Cancer Inst. 2013 Nov 6;105(21):1667-70). Fig. 3 shows the immunoreactivity of GluN2B at DG (Fig.3A), in mice treated or untreated with folate at dentate gyrus. Our results showed in folate-treated mice an increase in the expression of GluN2B immunoreactivity at the molecular layer, in comparison with mice injected with vehicle solution (Fig.3B).

### Effect of folate in extracellular matrix organization

Considering that extracellular matrix organized into perineuronal nets (PNN) are dynamic structure which increased with age, we checked possible reorganization of these structures due to folate effects. Results showed that folate injection induced in hippocampus a significant reduction in PNN density labeled with wisteria floribunda agglutinin (WFA) lectin in comparison with vehicle-injected aged mice (Fig.3C).

### 2.4. Impact of folate on methylation of histone and DNA in hippocampal neurons

Since the effect of folate goes behind adult neurogenesis and could be related to epigenetic changes, we have studied how folate may modify methylation in histones and DNA. Fig. 4 shows that the presence of folate after 14 days since its injection does not modify the level of methylation at H3K9me3 (Fig. 4 A,B) or H4K20me3 (Fig. 4 C,D) positions. However, when the level of 5-methycytosine (5mC) (Fig. 5A) was tested, significant changes for 5mc were observed in granular neuronal layer (P=0.0132) of dentate gyrus (DG) as well as an important tendency in the pyramidal layer of CA3 (P-value=0.05) and CA1 (P-value=0.06). Regarding 5-hydroxymethylcytosine (5hmc) not significant changes were found in hippocampal neuronal layers.

### 2.5. Possible mechanism for the folate action

Although, folate could act, in an indirectly way, as a methyl donor, something that methionine is doing in a direct way, folate could have additional mechanisms for its action. There is a folate cell receptor, folate receptor α (FRα) (Alam C, et al.,. Upregulation of reduced folate carrier by vitamin D enhances brain folate uptake in mice lacking folate receptor alpha. Proc Natl Acad Sci USA. 2019 Aug 27;116(35):17531-17540.), that upon binding to folate could be internalized into the cell nucleus acting as a transcription factor (Boshnjaku V, et al., Nuclear localization of folate receptor alpha: a new role as a transcription factor. Sci Rep. 2012;2:980. ). Since the presence of Frα could facilitate an increase in Oct4, Sox2 and klf4 expression in neural cells (Mohanty V, et al., (2016) Folate Receptor Alpha Upregulates Oct4, Sox2 and Klf4 and Downregulates miR-138 and miR-let-7 in Cranial Neural Crest Cells. Stem Cells 34: 2721-2732), and these factors have seen regulating the expression of DNA (cytosine-5)-methyltransferase 1 (Dnmt1) (Wu et al., 2018), we have tested if effects showed previously on 5-metyl cytosine DNA levels could be produced by an increase in Dnmt1 hippocampal expression. By western blot we have confirmed that folate injection increased the expression of Dnmt1 hippocampal levels (Fig. 6).

In view of above positive results about folate action mechanism through FRα, we decided to synthetized a peptide able to bind to this receptor to mimic folate effects and to verify that cognitive improvement seen by folate injection were induced mainly by FRa activation.

### 2.6 Potential reprogramming effects of FRα binding peptide

As previously indicated, it was reported that folate facilitates the expression of YF including Sox2. Thus, we have tested if FRa binding peptide (peptide of the invention having amino acid sequence SEQ ID NO: 1) has any effect on Sox2 expression. Fig. 9A shows that the presence of FRa binding peptide in aged mice resulted in a significant increase of SOX2 expression in different regions of dentate gyrus (Fig.9B), where injections were placed.

### 2.7 Cellular rejuvenation effects of FRα binding peptide on hippocampal region

### 2.7.1 FRα binding peptide effects on neuroplasticity marker GLUN2B

Since it has been previously described that the presence of YF, such as Sox2, could facilitate the expression of GluN2B, we have tested whether the presence of the binding peptide FRa (peptide of the invention having amino acid sequence SEQ ID NO: 1), which upregulates the expression of Sox2, it could also increase the expression of GluN2B. Fig. 10(A-C) shows that the presence of FRa peptide results in an increase of GLUN2B expression at the molecular layer of dentate gyrus (Fig.10C), like in the case of the presence of folate.

### 2.7.2 Effect of FRα binding peptide on extracellular matrix organization

Previously, it was shown that upon folate addition changes in extracellular matrix (EMC) organization takes place. Fig. 10 (D, E) shows a clear effect of the FRa binding peptide in the organization of extracellular matrix in a fashion similar to that found for the presence of folate, reducing the density of PNN in dentate gyrus.

Taken into account the results of Fig. 10, we can suggest an improvement in neuronal signaling by changes in the fraction of excitatory (glutamatergic) and inhibitory neurons. In addition, both results that could be related between them (glutamatergic receptors and PNN) since alterations in the expression of PNN components during aging, could be modulated by changes in NMDA function (Yamada J, et al., Alterations in expression of Cat-315 epitope of perineuronal nets during normal ageing, and its modulation by an open-channel NMDA receptor blocker, memantine. J Comp Neurol. 2017 Jun 15;525(9):2035-2049.).

### 2.8. Cognitive effects of FRα binding peptide

Intrahippocampal injection of this peptide (peptide of the invention having amino acid sequence SEQ ID NO: 1) in the same conditions of previous folate experiments, led to a significant improvement of recognition memory in aged mice only at long term. Not significant differences regarding vehicle-injected mice were found at short term (Fig. 7), reproducing same behavior results than with folate injections.

### 3. Discussion

It has been looked for a strategy to replace YF function, focused on reprogramming old cells to cells with younger features, by the use of more simple compounds. We have found that the addition of folate or FRa binding peptide could be a good replacement for Yamanaka Factors (YF). Firstly, folate treatment resulted in epigenetic changes related with the rejuvenation of methylation patterns as with YF. Also, an increase in the subunit GluN2B of NMDA receptor was found. This subunit is mainly present in young animals and this increase may suggest an improvement for synaptic plasticity, as previously described for YF expression (Rodríguez-Matellán A, et al., In Vivo Reprogramming Ameliorates Aging Features in Dentate Gyrus Cells and Improves Memory in Mice. Stem Cell Reports. 2020 Nov 10;15(5):1056-1066). This increased plasticity due to the transient folate or FRa presence could be behind the improvement in recognition and spatial memory which was found in these old mice. Interestingly, long-term but not short-term recognition memory was affected in these mice, as occurred with YF cyclical overexpression (Rodriguez-Matellan *et al.,* 2020). Furthermore, aging and age-associated diseases like Alzheimer disease in different brain regions including hippocampus, has been related with an increase of perineuronal nets (PNN) (Tanaka&Mizoguchi, Influence of aging on chondroitin sulfate proteoglycan expression and neural stem/progenitor cells in rat brain and improving effects of a herbal medicine, yokukansan. Neuroscience 164 (2009) 1224-1234)) which are a specific type of extracellular matrix wrap around some neurons which restrict their plasticity. These are dynamic structures which undergo changes throughout postnatal development and their reduction has been associated with a facilitation of neuronal plasticity and memory enhancement (Romberg et al., Depletion of perineuronal nets enhances recognition memory and long-term depression in the perirhinal cortex. J Neurosci;33(16):7057-65 (2013)). In this way, our results in aged mice showing the reorganization of extracellular matrix by reducing the density of perineuronal nets, would be replicating YF rejuvenating effects on hippocampus.

The mechanism for YF reprogramming could be the stimulation of DNA methyltransferase activity which consequently produce an increase in DNA methylation (Tsai et al., Oct4 and Nanog directly regulate Dnmt1 to maintain self-renewal and undifferentiated state in mesenchymal stem cells. Mol Cell 47: 169-182(2012)). Since DNA methylation could be also increased by the presence of methyl donors or cofactors that may facilitate DNA methylation, we have tested the possible reprogramming effect of folate and S-adenosylmethionine (SAM) as methyl donor and of vitamin B12 as a cofactor. Although methionine is the universal methyl donor, folate could act as a donor through the folate-methionine metabolism. On the other hand, even if methionine could be converted to SAM, SAM could also remethylate methionine through homocysteine ).

It's important to note that despite the involvement of both folate and SAM compounds in one carbon metabolism with similar functions, only folate was found to have a similar impact on cognition as with YF. That difference could be explained by its interaction with the folate receptor α (FRα), a transcription factor involved in the expression of the Yamanaka factor Oct4 (Mohanty V, et al., (2016) Folate Receptor Alpha Upregulates Oct4, Sox2 and Klf4 and Downregulates miR-138 and miR-let-7 in Cranial Neural Crest Cells. Stem Cells 34: 2721-27326), that could also increase the activity of DNMT1 (Wu F, et al., (2018) Oct4 regulates DNA methyltransferase 1 transcription by direct binding of the regulatory element. Cell Mol Biol Lett 23: 39 ). Our results support this hypothesis, considering the increased levels found of DNMT1 expression and of DNA methylation associated with heterochromatin regions. The same long-term recognition memory enhancement induced by FRa binding peptide treatment as with folate injections, endorse this hypothesis as well.

Folate deficiency related to aging may result in the hypomethylation of the DNA present in heterochromatin regions, after despirilization of chromatin ( Fenech M. Folate, DNA damage and the aging brain. Mech Ageing Dev 131: 236-241 (2010)). In addition, aging has been related with a global loss of DNA methylation, particularly at constitutively heterochromatin (Tsurumi A, Li WX. Global heterochromatin loss: a unifying theory of aging? Epigenetics. 2012 Jul;7(7):680-8), which potentially could lead to altered chromatin functioning and to a genomic instability (Hu, Z., et al., Nucleosome loss leads to global transcriptional up-regulation and genomic instability during yeast aging. Genes and Development, 28(4) (2014)).

In this way, it is well known that in the heterochromatin loss aging model, heterochromatin markers like DNA methylation decrease with age at the time of heterochromatin loss (Tsurumi A, Li WX. Global heterochromatin loss: a unifying theory of aging? Epigenetics. 2012 Jul;7(7):680-8).

Indeed, loss of heterochromatin has been found in premature aging disorders like Huntchinchon-Gilford progeria or Werner syndrome . Heterochromatin markers like DNA cytosine-5 methylation is globally decreased in aging at hippocampus (Szulwach K, et al., 5-hmC-mediated epigenetic dynamics during postnatal neurodevelopment and aging. Nat Neurosci 14(12):1607-16 (2011); Chen H, et al., Effect of aging on 5-hydroxymethylcytosine in the mouse hippocampus Restor Neurol Neurosci 30(3):237-45(2012)), leading to a general DNA hypomethylation (Ciccarone F, et al., DNA methylation dynamics in aging: how far are we from understanding the mechanisms? Mechanisms of Ageing and Development 174 3-17 (2018)). This hypomethylation may facilitate the presence of aging signatures, but if DNA methylation is promoted, those signatures may be corrected. In this way, rejuvenating effects of folate could be more related to its action like being a DNMT1 activator more than a methyl donor, just as Yamanaka Factors.

To conclude, the FRa binding peptide (the peptide of the invention), as folate, promote the rejuvenation of hippocampal region by increasing neuroplasticity. These effects could be promoted, or partially mediated, by the activation of DNMT-1 and the "re-methylation" of "aged" DNA (Fig. 8), improving cognitive function, something naturally deteriorated with aging.

## Claims

1. Peptide comprising an amino acid sequence with, at least, 75% of identity to amino acid sequence SEQ ID NO: 1, or a composition comprising said peptide, for use in treating and/or preventing a cognitive disorder in a subject.

2. Peptide or composition for use according to claim 1, wherein the peptide comprises amino acid sequence SEQ ID NO: 1.

3. Peptide or composition for use according to claim 1 or 2, wherein the cognitive disorder comprises a long-term memory impairment.

4. Peptide or composition for use according to any one of claims 1 to 3, wherein the cognitive disorder is selected from the list consisting of Alzheimer's disease, mild cognitive impairment (MCI), Huntington's disease, age-related long-term memory loss, FTLD, Korsakoff syndrome, vascular dementia, Lewy body dementia, progressive supranuclear palsy and any combination thereof.

5. Peptide or composition for use according to any one of claims 1 to 4, wherein the subject is a human.

6. Peptide or composition for use according to claim 5, wherein the subject is over 40 years old, preferably over 50 years, more preferably over 60 years, even more preferably over 70 years old.

7. Non-therapeutic use of a peptide comprising an amino acid sequence with, at least, 75% of identity to amino acid sequence SEQ ID NO: 1, or a composition comprising said peptide, for improving cognitive function in a subject.

8. Non-therapeutic use according to claim 7, wherein the peptide comprises amino acid sequence SEQ ID NO: 1.

9. Non-therapeutic use according to claim 7 or 8, wherein the improvement of the cognitive function comprises an amelioration of the subject long-term memory.

10. Non-therapeutic use according to any one of claims 7 to 9, wherein the cognitive function is selected from the list consisting of learning, memory, complex attention, executive functioning and any combination thereof.

11. Non-therapeutic use according to any one of claims 7 to 10, wherein the subject is a human.

12. Non-therapeutic use according to claim 11, wherein the subject is over 40 years old, preferably over 50 years, more preferably over 60 years, even more preferably over 70 years old.
